Europäisches Patentamt

European Patent Office

Office européen des brevets

(19)

(11) Publication number: **0 300 459**
**A2**

# EUROPEAN PATENT APPLICATION

(12)

(21) Application number: 88111704.8

(22) Date of filing: 20.07.88

(51) Int. Cl.4 **C07K 7/10 , A61K 37/64 ,**
**C12N 15/00 , C12P 21/02 ,**
**C12N 1/20**

(30) Priority: 23.07.87 JP 184556/87

(43) Date of publication of application:
**25.01.89 Bulletin 89/04**

(84) Designated Contracting States:
**AT BE CH DE ES FR GB GR IT LI NL SE**

(71) Applicant: MOCHIDA PHARMACEUTICAL CO.,
LTD.
7, Yotsuya 1-chome
Shinjuku-ku Tokyo 160(JP)

(72) Inventor: Nobuhara, Masahiro
572-10, Ohaza-Yajuro
Koshigaya-shi Saitama(JP)
Inventor: Kanamori, Toshinori
3556-5, Ohaza-Minamiogishima
Koshigaya-shi Saitama(JP)
Inventor: Ogino, Hiromi
10-5-502, Horikiri 8-chome
Katsushika-ku Tokyo(JP)
Inventor: Mochida, Ei
5-4, Komagome 2-chome
Toshima-ku Tokyo(JP)

(74) Representative: Henkel, Feiler, Hänzel &
Partner
Möhlstrasse 37
D-8000 München 80(DE)

(54) Human pancreatic secretory trypsin inhibitor.

(57) A human pancreatic secretory trypsin inhibitor (PSTI) essentially free of other proteins of human origin is produced by using recombinant DNA techniques. The human PSTI is prepared by transforming E. coli into a transformant capable of producing the human PSTI, cultivating the transformant to produce the human PSTI, and recovering the human PSTI from the culture supernatant. The transformant may preferably be prepared by transforming a cell wall lipoprotein-deleted mutant E. coli strain by using a vector replicable in E. coli. The vector may preferably comprise a DNA sequence coding for the human PSTI, as well as a promotor, an SD sequence, and a DNA sequence coding for a signal peptide. The present invention also relates to a process for producing the human PSTI, a vector useful in the process, and an E. coli transformant having the vector.

EP 0 300 459 A2

## Human Pancreatic Secretory Inhibitor

### BACKGROUND OF THE INVENTION

This invention relates to a useful protein produced by recombinant DNA technology, and particularly to a human pancreatic secretory trypsin inhibitor. This invention also relates to a process for producing said protein, a recombinant vector including a DNA sequence encoding said protein, and a transformant of E. coli including said vector.

A pancreatic secretory trypsin inhibitor (hereinafter abbreviated as PSTI) is a known protein secreted into pancreatic juice, and has a trypsin inhibiting activity (J. Am. Chem. Soc., vol. 70, 3034-3040,1948). The PSTI is also known to be found in human blood, where it is detected by radioimmunoassay (hereinafter abbreviated as RIA) using a polyclonal antibody and a radioactive substance. An exemplary RIA system is PSTI Test-Shionogi manufactured by Shionogi & Co.,Ltd.

A stable production of an anti-human PSTI monoclonal antibody would be enabled if the human PSTI is obtained and a hybridoma producing a monoclonal antibody against the human PSTI is prepared therefrom. The monoclonal antibody would in turn increase a detection sensitivity of the human PSTI when used in the measurement of the human PSTI concentration. The measurement of human PSTI utilizing the monoclonal antibody, therefore, will enable a quite simple measurement without using any radioactive substances.

The amount of human PSTI in blood is increased during acute pancreatitis Tan to Sui, (Cholesystis and Pancreas) vol. 3, 383-388,1982). Therefore, the human PSTI, when isolated in a commercial scale, is expected to realize an easier diagnosis of various PSTI-associated deseases, including the acute pancreatitis. It is, however, quite difficult to obtain a large amount of contaminant-free human PSTI by extracting and purifying from human pancreatic juice or blood which are not readily available. An improvement is thus desired in techniques for producing the human PSTI.

In recent years, recombinant DNA techniques have been developed utilizing prokaryotic cells such as E. coli(Esherichia coli), yeasts, mammalian cell lines, and the like as hosts. Among these, the systems utilizing E. coli as their host are most widely used in industrial productions since E. coli is easy to cultivate, E. coli can be cultivated at low cost, and E. coli has a high production potential.

The system using E. coli as its host, however, has several problems including (1) the protein produced has methionine residue at its N terminal, (2) the protein produced may become toxic to E. coli itself when a large amount of the protein is accumulated within the cell, and (3) the protein produced within E. coli may not be recovered if it is unstable within the cell.

A technique is also known in which proteins produced within E. coli are transported through cytoplasmic membrane to accumulate in the periplasm. To recover the thus accumulated protein in the periplasm, an osmotic shock of the cells must be carried out to direct the desired protein into the exterior of the cells after a centrifugation to separate the cells from the medium.

These processes are quite disadvantageous in terms of purifying the desired protein since the desired protein is likely to be contaminated by other proteins from E. coli.

Itakura et al. discloses that low molecular-weight proteins produced by recombinant DNA techniques utilizing E. coli as the host are susceptible to be degradated by proteolytic enzymes within E. coli (Science, vol.198, 1056-1063,1977). No techniques has been known to produce a large amount of low molecular-weight proteins such as the human PSTI having a molecular weight of 6 kilodalton (hereinafter abbreviated as kd) in a form having an identical amino acid sequence as its natural form at low cost by utilizing E. coli as the host. It is, therefore, desired to develop a recombinant DNA technique capable of efficiently producing a large amount of the desired low molecular-weight protein.

It is a main object of the present invention to provide a useful protein, particularly the human PSTI. Another object of the present invention is to provide a process for producing the human PSTI, particularly by utilizing recombinant DNA techniques. and a process for efficiently recovering the human PSTI from the host E. coli. A further object of the present invention is to provide a vector useful in carrying out said process for producing the human PSTI, and a microorganism which has been transformed by said vector.

## SUMMARY OF THE INVENTION

According to the present invention, there is provided a human PSTI produced by using recombinant DNA techniques, a process for producing the human PSTI by using recombinant DNA techniques and efficiently recovering the thus produced human PSTI from the host E. coli, a recombinant vector useful in said process, and an E. coli transformant by said vector.

According to a first aspect of the present invention, there is provided a human PSTI essentially free of other proteins of human origin.

The human PSTI is preferably prepared by transforming E. coli into a transformant capable of producing the human PSTI, and cultivating the transformant to produce the human PSTI.

The transformant may preferably be E. coli having a vector replicable in E. coli and including a DNA sequence encoding human PSTI.

The vector may preferably comprise both

(A) a DNA sequence encoding the human PSTI, and

(B) a promoter, an SD sequence, and a DNA sequence encoding a signal peptide, which function within E. coli.

The vector may preferably be a plasmid designated pM463.

The E. coli may preferably have a mutation on its cell wall.

The E. coli may preferably be a strain designated JE5505 or JE5513.

The human PSTI may preferably be a protein comprising an amino acid sequence selected from

(a) Asp-Ser-Leu-Gly-Arg-Glu-Ala-Lys-Cys-Tyr-Asn-Glu-Leu-Asn-Gly-Cys-Thr-Lys-Ile-Tyr-Asp-Pro-Val-Cys-Gly-Thr-Asp-Gly-Asn-Thr-Tyr-Pro-Asn-Glu-Cys-Val-Leu-Cys-Phe-Glu-Asn-Arg-Lys-Arg-Gln-Thr-Ser-Ile-Leu-Ile-Gln-Lys-Ser-Gly-Pro-Cys,

(b) Asp-Ser-Leu-Gly-Arg-Glu-Ala-Lys-Cys-Tyr-Asn-Glu-Leu-Asn-Gly-Cys-Thr-Lys-Ile-Tyr-Asp-Pro-Val-Cys-Gly-Thr-Asp-Gly-Asn-Thr-Tyr-Pro-Asn-Glu-Cys-Val-Leu-Cys-Phe-Glu-Asn-Arg-Lys-Arg-Glu-Thr-Ser-Ile-Leu-Ile-Gln-Lys-Ser-Gly-Pro-Cys,

(c) Asp-Ser-Leu-Gly-Arg-Glu-Ala-Lys-Cys-Tyr-Asn-Glu-Leu-Asn-Gly-Cys-Thr-Lys-Ile-Tyr-Asp-Pro-Val-Cys-Gly-Thr-Asp-Gly-Asn-Thr-Tyr-Pro-Asn-Glu-Cys-Val-Leu-Cys-Phe-Glu-Asn-Arg-Lys-Arg-Gln-Thr-Ser-Ile-Leu-Ile-Glu-Lys-Ser-Gly-Pro-Cys, and

(d) Glu-Ala-Lys-Cys-Tyr-Asn-Glu-Leu-Asn-Gly-Cys-Thr-Lys-Ile-Tyr-Asp-Pro-Val-Cys-Gly-Thr-Asp-Gly-Asn-Thr-Tyr-Pro-Asn-Glu-Cys-Val-Leu-Cys-Phe-Glu-Asn-Arg-Lys-Arg-Gln-Thr-Ser-Ile-Leu-Ile-Gln-Lys-Ser-Gly-Pro-Cys.

According to a second aspect of the present invention, there is provided a process for producing a human PSTI comprising transforming E. coli into a transformant capable of producing the human PSTI, cultivating said transformant to produce the human PSTI, and recovering the human PSTI from the culture supernatant.

According to a third aspect of the present invention, there is provided a vector replicable in E. coli comprising

(A) a DNA sequence encoding the human PSTI, and

(B) a promoter, an SD sequence, and a DNA sequence encoding a signal peptide, which function within E. coli.

According to a fourth aspect of the present invention, there is provided a transformant of E. coli including a vector replicable in E. coli comprising

(A) a DNA sequence encoding the human PSTI, and

(B) a promoter, an SD sequence, and a DNA sequence encoding a signal peptide, which function within E. coli.

3

## BRIEF DESCRIPTION OF THE DRAWINGS

FIG. 1 shows (1 ) a 204 bp DNA sequence coding for a SD region of lambda phage cII and the human PSTI isoinhibitor B, and (2) a translation-initiation amino acid methionine and an amino acid sequence of the human PSTI isoinhibitor B coded by said DNA sequence.

FIG. 2 shows a construction of plasmid pM450.

FIG. 3a shows a construction of plasmid pM463.

FIG. 3b shows (1) a DNA sequence incorporated into the plasmid pM463 coding for the amino acid sequence of the protein comprising an alkaline phosphatase signal peptide linked with the human PSTI isoinhibitor B, and (2) an amino acid sequence of the protein coded by said DNA sequence.

FIG. 4 is a diagram illustrating a time-course of the amount of the human PSTI secreted to the culture supernatant and the periplasm fraction.

FIG. 5 illustrates an immunological identification of the human PSTI produced by the transformant, and immunological identification and protein staining of the purified human PSTI subsequent to an electrophoresis.

FIG. 6 is a scheme illustrating the purification of the human PSTI produced by the transformant.

FIG. 7 shows an elution profile in a reverse-phase HPLC of the human PSTI produced in the transformant.

FIG. 8 is a diagram illustrating the trypsin inhibition activity of the human PSTI produced in the transformant.

## DETAILED DESCRIPTION OF THE INVENTION

The present invention is hereinafter described in detail.

In the following description, nucleotides, amino acids, and peptides may be expressed in their abbreviations, which are commonly used in the art. The abbreviations used herein are:

DNA: deoxyribonucleic acid,
RNA: ribonucleic acid,
cDNA: complementary DNA,
mRNA: messenger RNA,
A: adenine,
T: thymine,
G: guanine,
C: cytosine,
EDTA: ethylenediaminetetraacetic acid,
SDS: sodium dodecyl sulfate,
Ala: alanine,
Arg: arginine,
Asn: asparagine,
Asp: aspartic acid,
Asx: asparagine or aspartic acid,
Cys: cysteine,
Gln: glutamine,
Glu: glutamic acid,
Glx: glutamine or glutamic acid,
Gly: glycine,
His: histidine,
Ile: isoleucine,
Leu: leucine,
Met: methionine,
Phe: phenylalanine,
Pro: proline
Ser: serine
Thr: threonine

4

Trp: tryptophan,
Tyr: tyrosine,
Val: valine, and
Lys: lysine.


(A) Human PSTI gene

Human PSTI is a polypeptide wherein at least four known molecule species, that is, A₁, A₂, B and C are present. Amino acid sequences of these four isoinhibitors are known (J. Biochem., vol. 98, 687-694, 1985), and cDNA encoding at least one of the isoinhibitors has also been isolated (Biochem. Biophys. Commun., vol.132, 605-612, 1985). Therefore, a DNA sequence encoding the human PSTI may be obtained by extracting a genomic DNA of a human cell, by synthesizing a human PSTI cDNA from a mRNA extracted from a human cell, or by designing the DNA sequence encoding the human PSTI on the basis of the amino acid sequence of any of the four isoinhibitors and chemically synthesizing the DNA according to said design.

The DNA sequences encoding the human PSTI are those encoding the four amino acid sequences (a), (b), (c), and (d) as shown below, which correspond to the human PSTI isoinhibitors B, A₁, A₂, and C, respectively. The genes encoding modified human PSTI obtained by substitution, deletion or insertion of the amino acid sequence are also within the scope of the present invention.

(a)
Asp-Ser-Leu-Gly-Arg-Glu-Ala-Lys-Cys-Tyr-Asn-Glu-Leu-Asn-Gly-Cys-Thr-Lys-Ile-Tyr-Asp-Pro-
Val-Cys-Gly-Thr-Asp-Gly-Asn-Thr-Tyr-Pro-Asn-Glu-Cys-Val-Leu-Cys-Phe-Glu-Asn-Arg-Lys-
Arg-Gln-Thr-Ser-Ile-Leu-Ile-Gln-Lys-Ser-Gly-Pro-Cys,

(b)
Asp-Ser-Leu-Gly-Arg-Glu-Ala-Lys-Cys-Tyr-Asn-Glu-Leu-Asn-Gly-Cys-Thr-Lys-Ile-Tyr-Asp-Pro-
Val-Cys-Gly-Thr-Asp-Gly-Asn-Thr-Tyr-Pro-Asn-Glu-Cys-Val-Leu-Cys-Phe-Glu-Asn-Arg-Lys-
Arg-Glu-Thr-Ser-Ile-Leu-Ile-Gln-Lys-Ser-Gly-Pro-Cys,

(c)
Asp-Ser-Leu-Gly-Arg-Glu-Ala-Lys-Cys-Tyr-Asn-Glu-Leu-Asn-Gly-Cys-Thr-Lys-Ile-Tyr-Asp-Pro-
Val-Cys-Gly-Thr-Asp-Gly-Asn-Thr-Tyr-Pro-Asn-Glu-Cys-Val-Leu-Cys-Phe-Glu-Asn-Arg-Lys-
Arg-Gln-Thr-Ser-Ile-Leu-Ile-Glu-Lys-Ser-Gly-Pro-Cys, and

(d)
Glu-Ala-Lys-Cys-Tyr-Asn-Glu-Leu-Asn-Gly-Cys-Thr-Lys-Ile-Tyr-Asp-Pro-Val-Cys-Gly-Thr-Asp-
Gly-Asn-Thr-Tyr-Pro-Asn-Glu-Cys-Val-Leu-Cys-Phe-Glu-Asn-Arg-Lys-Arg-Gln-Thr-Ser-Ile-Leu-
Ile-Gln-Lys-Ser-Gly-Pro- Cys.

An illustrative example of the DNA sequences encoding the amino acid sequence (a) is:
GAC TCC CTG GGA AGA GAG GCC AAA TGT TAC AAT GAA CTT AAT GGA TGC ACC AAG
ATA TAT GAC CCT GTC TGT GGG ACT GAT GGA AAT ACT TAT CCC AAT GAA TGC GTG
TTA TGT TTT GAA AAT CGG AAA CGC CAG ACT TCT ATC CTC ATT CAA AAA TCT GGG
CCT TGC

Such a DNA sequence may readily be converted to a plurality of other sequences coding for the same amino acid sequence since most amino acids correspond to more than one codon. An illustrative example of such DNA sequences is given below, which is also within the scope of the present invention.
GAC TCC CTA GGT CGC GAG GCC AAA TGT TAC AAT GAA CTT AAT GGA TGC ACC AAG
ATA TAT GAC CCT GTC TGT GGG ACT GAT GGA AAT ACT TAT CCC AAT GAA TGC GTG
TTA TGT TTT GAA AAT CGG AAA CGC CAG ACA TCG ATC CTC ATT CAA AAA TCT GGG
CCT TGC

The chemical synthesis of the DNA may be carried out by designing the desired DNA sequence, optionally dividing the sequence into appropriate fragments, and chemically synthesizing the oligodeoxy-ynucleotides corresponding to said fragments by using a fully automated DNA chemical synthesizer, for example, Model 380A manufactured by Applied Biosystems Inc. The thus obtained oligodeoxynucleotide

5

may optionally be phosphorylated at its 5′ end by T4 polynucleotide kinase followed by annealing, and ligating by T4 DNA ligase. The thus obtained DNA is cloned into an appropriate vector.

The human PSTI gene may also be prepared from natural substances by either synthesizing cDNA or by isolating from genomic DNA.

The cDNA may typically be synthesized by isolating a mRNA from a human tissue or cell wherein the human PSTI mRNA are transcribed, by means of a known process, for example, as described in T. Maniatis et al. ed., "Molecular Cloning, a Laboratory Manual", Cold Spring Harbor Laboratory,1982; and then synthesizing the cDNA by using the mRNA as a template by a known process. Various processes are known for the cDNA synthesis such as those utilizing S1 nuclease as described in "Molecular Cloning, a Laboratory Manual", the method of Land as described in Nucleic Acid Research, vol. 9, 2251-2266,1981, and the method of Okayama and Berg as described in Mol. Cell. Biol. vol. 2, 161-170, 1982. The thus synthesized cDNA is then cloned into plasmids or phages. The plasmid or phage having the human PSTI gene inserted thereto is detected by hybridization using a DNA or an RNA complementary to said cDNA of the human PSTI. The human PSTI gene may be isolated from the thus prepared plasmid or phage.

The human PSTI gene may also be obtained from a genomic DNA. This process is carried out by extracting a genomic DNA from the a tissue or cell, fragmenting the genomic DNA by appropriate restriction endonucleases or by certain physical means, cloning the fragments into plasmids or a phages, and detecting and isolating the human PSTI gene by hybridization as described above by using a DNA or an RNA complementary to said cDNA of the human PSTI.

(B) Promoter, Shine-Dargarno sequence, and signal peptide gene

Any promoter and Shine-Dargarno (SD) sequence capable of functioning within E. coli may be utilized as a promoter and SD sequence for expressing the human PSTI in E. coli. Typical promoters which may be used herein include tryptophan operon promoter, $P_R$ promoter of lambda phage, lactose operon promoter, and $P_L$ promoter of lambda phage. Typical SD sequences which may be used herein include tryptophan operon SD sequence, cII SD sequence of lambda phage, and lipoprotein gene-SD sequence.

Any signal peptide coding sequences capable of allowing a permeation of the human PSTI expressed within E. coli cell through cytoplasmic membrane may be utilized in the present invention to facilitate the secretion of the human PSTI into the exterior of the cytoplasm. The signal peptide coding sequence may preferably be the DNA sequence coding for signal peptide of alkaline phosphatase, β-lactamase, lipoproteins, or outer membrane proteins.

The DNA sequences of the promoter, the SD region or the signal peptide may be obtained either from a chromosomal DNA of E. coli, plasmid DNA or phage DNA, or through a chemical synthesis.

It is to be noted that partially modified promoters and SD sequences also have similar functions, and these partially modified promoters and SD sequences may also be utilized in carrying out the present invention. Also, those skilled in the art will readily be able to prepare a plurality of DNA sequences encoding the same amino acid sequence since most amino acids correspond to more than one codon. In addition, those skilled in the art will readily expect that modified natural signal peptides wherein a part of their amino acid sequence is converted to another sequence may still retain their function as a signal peptide. Therefore, sequences corresponding to such modified signal peptides are also suitable for use herein.

(C) Human PSTI-expression vector

Human PSTI-expression vector may be any vector which is replicable in E. coli and which essentially comprises the promoter, the SD sequence, the signal peptide gene, and the human PSTI gene. The vector may either be a natural vector or an entirely or partly synthesized vector. The sequence corresponding to the part of the human PSTI-expression vector serving the function of replication may preferably be derived from a plasmid capable of reproducing a large number of copies. Particularly preferred are the sequences serving the replication function in plasmids pBR322 and pACYC184.

(D) Host

Host transformed with the human PSTI-expression vector may be any E. coli as long as it satisfies the requirements that it is capable of replicating the human PSTI-expression vector and that it is capable of expressing the human PSTI coded in the human PSTI-expression vector. Preferred E. coli strains include HB101, C600, W3110, SK107, and SG4044 strains, and those having a mutation on their cell walls, such as JE5505 and JE5513 strains. Most preferred are JE5505 and JE5513 strains.

"The E. coli having a mutation on its cell wall" herein designates the E. coli having a cell wall which allows an easy permeation of proteins therethrough. Typical examples include lipoprotein-deleted mutant strains, that is, the mutant strains lpo⁻ or lpp⁻ wherein the lipoprotein of the outer membrane constituting the cell wall is deleted (Molec. gen. Genet., vol. 167,1-9, 1978), among which JE5505 and JE5513 strains of E. coli being preferred.

Various processes have been tried to facilitate the secretion of the proteins produced within the cytoplasm into the exterior of the cytoplasm by taking an advantage of the signal sequence which participate in the permeation through the cytoplasmic membrane of the proteins when the proteins produced are toxic to E. coli itself or susceptible to be degradated in the cytoplasm. E. coli, however, is a Gram-negative bacteria, and the proteins produced in the cytoplasm and permeated through the cytoplasmic membrane is accumulated in the periplasm characteristic to such a Gram-negative cell rather than being secreted into the exterior of the cell. The proteins accumulated in the periplasm are then susceptible to be degradated by various proteases present in the periplasm.

On the other hand, there are mutant strains of E. coli (lpo⁻ or lpp⁻) wherein the outer membrane constituting the cell wall has a mutation and the lipoprotein is lacking from the outer membrane, as mentioned above. In such a mutant strain, substances like proteins are more easily transported through the cell wall since a component constituting the cell wall is absent therefrom. Therefore, when the technique for transporting xenogenic proteins into the periplasm is combined with a host of such a lipoprotein deleted E. coli by means of the recombinant DNA techniques, a secretion of the desired protein into the culture medium will be realised at a high rate enabling a recovery of the desired protein from the medium.

(E) Cultivation

Cultivation may be carried out in accordance with the conventional processes used for cultivating microoganisms by consulting various publications, such as "Laboratory Methods in Microbiology", W. F. Harrigan et al. ed., Academic Press,1966, and "Seibutsukagakukogaku", S. Aiba et al. ed., Tokyo Daigaku Shuppankai,1976.

Conventional culture media may be used for cultivating E. coli, for example, L broth and M9CA medium may be used in the present invention at a temperature at which E. coli can propagete or grow. E. coli may preferably be cultivated either in a shaking culture or in a jar fermenter, although other cultivation process may be adopted for producing the desired protein.

(F) Purification of human PSTI

Purification of the thus produced human PSTI may be carried out by various known purification processes disclosed in such a publication as "Seikagaku Jikken Kouza 1, Tanpakushitsu no kagaku", Japan Biochemistry Society ed., Tokyo Kagaku Dojin,1976. The purification process may be selected from salting-out, dialysis, ion-change chromatography, affinity chromatography, gel chromatography, high performance liquid chromatography, electrophoresis, and the combination thereof.

(G) Human PSTI

The human PSTI according to the present invention may be produced either by a microorganism culture system or in a cell line culture system, and have a biologically active form corresponding to the natural human PSTI, and be free of other proteins of human origin, and optionally be secreted into the exterior of the cytoplasm of the host E. coli.

Therefore, contaminants originating from the host are already minimal before undergoing the purification stage. The human PSTI obtained in the present invention is thus free of any contaminants generally associated with the purified human PSTI from natural sources.

## EXAMPLES

In order that those skilled in the art will be better able to practice the invention, the following examples are given by way of illustration and not by way of limitation.

Experiments in the present invention were carried out in accordance with "Guideline for Recombinant DNA Experiments" established by the Prime Minister of Japan. In the examples, the plasmids, DNA, enzymes, E. coli, and the like were treated by consulting the following publications.

1. Proteins, Nucleic Acids and Enzymes, vol. 26, special issue "gene manipulation", Kyoritsu Shuppan K.K., 1981.

2. "Idenshisousa Jikken-hou", Y. Takagi ed., Kodansha K.K., 1980.

3. "Idenshisousa Manual", Y. Takagi ed., Kodansha K.K., 1980.

4. "Molecular Cloning, a Laboratory Manual", T. Maniatis et al. ed., Cold Spring Harbor Laboratory.1982.

5. "Methods in Enzymology" vol. 65, L. Grossman et al. ed., Academic Press,1980.

6. "Methods in Enzymology" vol. 68, R. Wu ed., Academic Press,1979.

In the examples, the human PSTI designates the human PSTI isoinhibitor B unless otherwise specified.

Example 1 Preparation of human PSTI-expression vector, and preparation of E. coli strain capable of expressing human PSTI

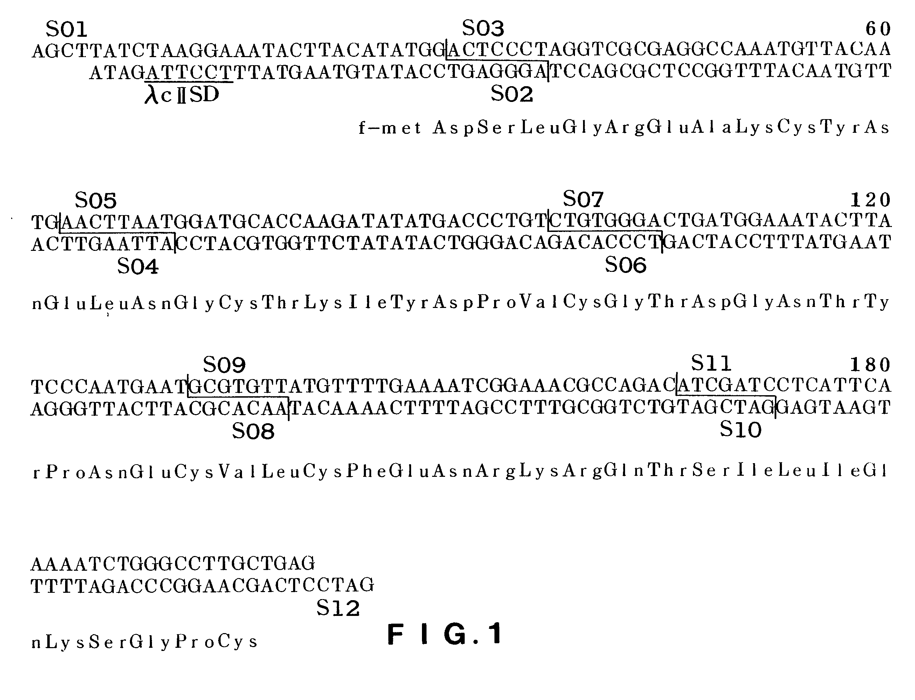

A chemically synthesized DNA including the human PSTI gene was prepared as set forth below. A 204 bp DNA sequence comprising a sequence corresponding to the amino acid sequence of the human PSTI isoinhibitor B (J. Biochem., vol. 98, 687-694, 1985) and a sequence encoding the SD sequence of the lambda phage cII was designed, and this DNA sequence was divided into 12 oligodeoxynucleotides S01 to S12, 6 for each of the strand (FIG. 1). Using a fully automatic DNA synthesizer (Model 380, manufactured by Applied Biosystems Inc.), 12 oligodeoxynucleotides were chemically synthesized, and purified by high performance liquid chromatography (hereafter abbreviated as HPLC) using a reverse phase column. S01 and S12 were phosphorylated by T4 polynucleotide kinase. S01 and S12 were mixed with the remaining 10 oligomers S02 to S11, and assembled together by annealing to constitute the 204 bp double-stranded DNA fragment.

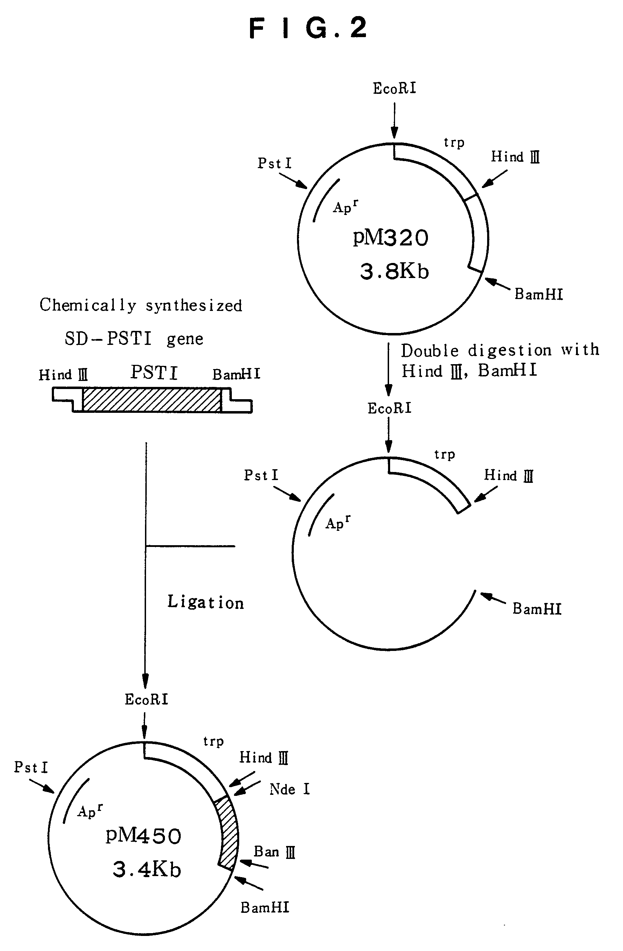

The plasmid pM320 is a derivative of the plasmid pBR322, and is replicabile in E. coli and has an ampicillin resistant gene and tryptophan promoter therein (Nucleic Acid Research Symposium Series, vol. 17, 131-134, 1986, and FIG. 2). The plasmid pM320 was digested with restriction endonucleases HindIII and BamHI, and the resulting DNA fragment mixture was subjected to an electrophoresis using a 0.7% agarose gel. An approximately 3.2 kbp DNA fragment was adsorbed on diethylaminoethyl cellulose paper (hereinafter abbreviated as DEAE cellulose paper) to separate from an approximately 0.6 kbp DNA fragment. The DEAE cellulose paper was then washed with a high-concentration salt solution (2M NaCl/10mM Tris-HCl, pH 7.5/1mM EDTA) to recover the approximately 3.2 kbp DNA fragment from the DEAE cellulose paper. A series of such process is hereinafter referred to as a DNA isolation by using a DEAE cellulose paper.

The above-described 204 bp double-stranded fragment and the approximately 3.2 kbp DNA fragment derived from pM320 were assembled by using T4 DNA ligase, and the resulting DNA was used to transform the E. coli strain HB101. Ampicillin resistant colonies were isolated to obtain the desired transformant. A plasmid DNA was isolated from the thus obtained transformant, and was designated pM450 (FIG. 2). The transformed E. coli strain HB101 was designated pM450/HB101.

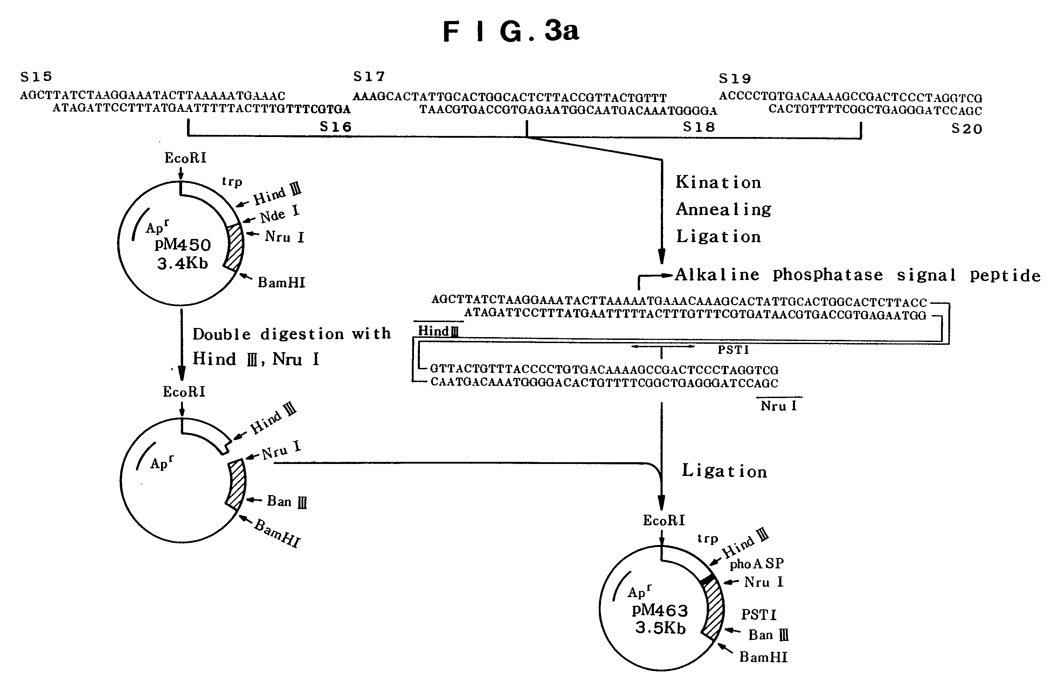

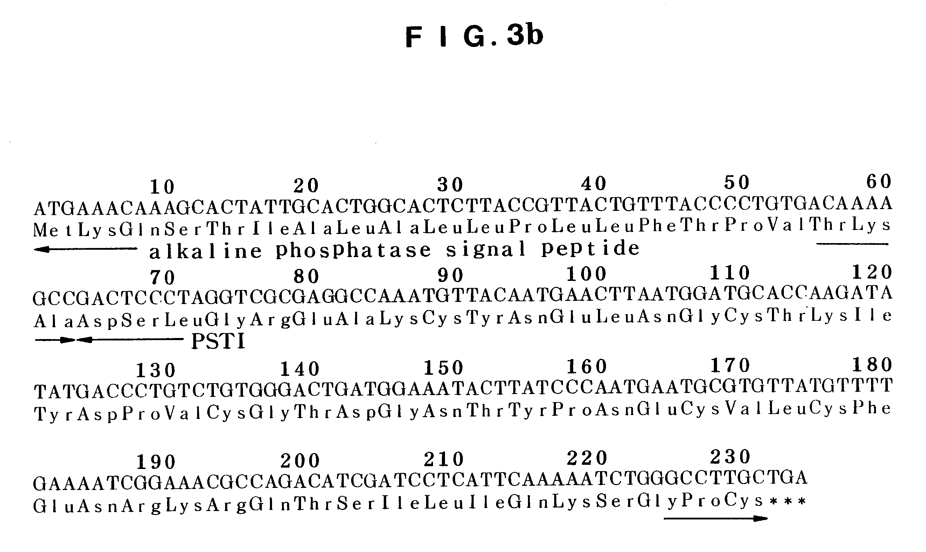

The human PSTI expression vector was prepared as described below. A 102 bp DNA sequence comprising a sequence coding for the SD sequence of the lambda phage cII ("Lambda II", R. W. Hendrix ed., Cold Spring Harbor Laboratory, 1983), a sequence coding for 21 amino acids of the alkaline phosphatase signal peptide (Nucleic Acid Research, vol. 9, 5671-5678, 1981), and a sequence coding for amino acids on the amino terminal of the human PSTI was designed, and this DNA sequence was divided into 6 oligodeoxynucleotides S15 to S20, 3 for each of the strand (FIGS. 3a and 3b). Each oligodeoxynucleotide was chemically synthesized, purified, and recovered in a similar manner as described above. S15 and S20 were also phosphorylated. These 2 oligodeoxynucleotides were mixed with the remaining 4 oligomers, and assembled together by annealing to constitute the 102 bp double-stranded DNA.

In the meantime, pM450 (FIG. 2) was digested with restriction endonucleases HindIII and NruI. Of the resulting DNA fragments, an approximately 3.4 kbp DNA fragment was isolated by the above-described process utilizing the DEAE cellulose paper.

The above-described 102 bp double-stranded fragment and the approximately 3.4 kbp DNA fragment derived from pM450 were assembled by using T4 DNA ligase, and the resulting DNA was used to transform E. coli strain HB101. Ampicillin resistant colonies were isolated to obtain the desired transformant. A plasmid DNA was isolated from the thus obtained transformant, and was designated pM463 (FIG.3a). The transformed E. coli strain HB101 was designated pM463/HB101.

Example 2 Preparation of E. coli strain capable of expressing human PSTI

E. coli strain JE5505, a lipoprotein-deleted mutant (lpo⁻) (Molec. gen. Genet., vol.168,1-9,1978) was made into a competent cell by a known method (DNA Cloning, vol. 1, a practical approach, D. M. Glover ed., 109-135, IRL Press, 1985), and used for transformation with the plasmid pM450 prepared in Example 1. Ampicillin resistant colonies were selected, and the thus obtained transformant was designated pM450/JE5505.

E. coli strain JE5505 was similarly transformed with the plasmid pM463 prepared in Example 1, and the thus obtained transformant was designated pM463/JE5505 (Fermentation Research Institute Agency of Industrial Science and Technology deposit No.1949(FERM BP-1949) by Budapest Treaty on the International Recognition of the Deposit of Microorganisms for the Purposes of Patent Procedure).

Example 3 Production of human PSTI

(1) Expression of human PSTI

Single colony for each of the purified pM450/HB101, pM463/HB101, pM450/JE5505, and pM463/JE5505 strains was innoculated into 5 ml of 100 μg/ml ampicillin-containing L broth (containing 10 g bactotryptone, 5 g yeast extract, 1 g glucose, and 5 g sodium chloride per liter) and grown overnight at 37°C under shaking. The culture medium was diluted with 50-fold volume of the 100 μg/ml ampicillin-containing L broth, and grown at 37°C under shaking until it reached its late-logarithmic growth phase. The culture medium was centrifuged at 6,000 rpm for 5 minutes by a centrifuge 05PR-22 manufactured by Hitachi Koki K.K. and the pellet was resuspended in an equal volume as the L broth of 100 μg/ml ampicillin-containing M9CA medium (containing 15 g/l $Na_2HPO_4 \bullet 12H_2O$, 3 g/l $KH_2PO_4$, 0.5 g/l NaCl, 1 g/l $Na_4Cl$, 11 mg/l $CaCl_2$, 0.5 g/l $MgSO_4 \bullet 7H_2O$, 5 g/l Casamino acid, 10 g/l glucose, and 5 mg/l vitamin $B_2$). After cultivating at 37°C for 1 hour under shaking, 3-indoleacrylic acid was added to the culture medium to a final concentration of 10 μg/ml and it was further cultivated overnight.

The culture medium was divided into three fractions, namely, culture supernatant, periplasm, and cytoplasm fractions in accordance with a known method (Proc. Natl. Acad. Sci. USA, vol. 78, 5401-5405, 1981). The amount of the human PSTI in these three fractions were measured by RIA. The amounts of the human PSTI in the culture supernatant, the periplasm fraction, and the cytoplasm fraction are shown in Table 1, Experiment 1, as a value per 1 ml medium.

1.4 and 5.9 μg/ml of human PSTI were found in the supernatant fractions of pM463/HB101 and pM463/JE5505 strains, 0.37 and 0.031 μg/ml were found in the periplasm fractions of pM463/HB101 and pM463/JE5505 strains, and 0.003, 0.002 and 0.55 μg/ml were found in the cytoplasm fractions of pM450/HB101, pM450/JE5505 and pM463/JE5505 strains, respectively. No human PSTI was found in the cytoplasm fraction of pM463/HB101 strain and the supernatant strains of pM450/HB101 and pM450/JE5505 strains.

Further, pM463/HB101 and pM463/JE5505 strains were cultivated in a similar manner as described above except for that isopropyl β-D-thiogalactopylanoside was added to the culture to final concentration of 2mM simultaneously with the 3-indoleacrylic acid.

The culture medium was divided into three fractions, namely, culture supernatant, periplasm, and cytoplasm fractions. The amount of the human PSTI was measured as described above, and $\beta$-galactosidase activity was measured in a known method (Method in Enzymology, vol. 1, S. P. Colowick et al. ed.. 241-228. Academic Press, 1955). The results are shown in Table 1, Experiment 2.

1.5 and 31 $\mu$g/ml of the human PSTI were found in the supernatant fractions, 0.25 and 0.8 $\mu$g/ml were found in the periplasm fractions, and 0.04 and 0.8 $\mu$g/ml were found in the cytoplasm fractions of pM463/HB101 and pM463/JE5505 strains, respectively.

The measurement of the $\beta$-galactosidase activity carried out along with the measurement of the human PSTI revealed that, in pM463/HB101 and pM463/JE5505 strains, most of the $\beta$-galactosidase activity expressed was found in the cytoplasm fraction, and only 4% and 1% of the total activity detected was present in the periplasm fraction and the supernatant, respectively.

$\beta$-galactosidase is essentially an enzyme contained in the cytoplasm of the host E. coli. The low $\beta$-galactosidase activity found in the culture supernatant, therefore, indicates that Cells were little lysed in the course of the cultivation and the subsequent fractionation.

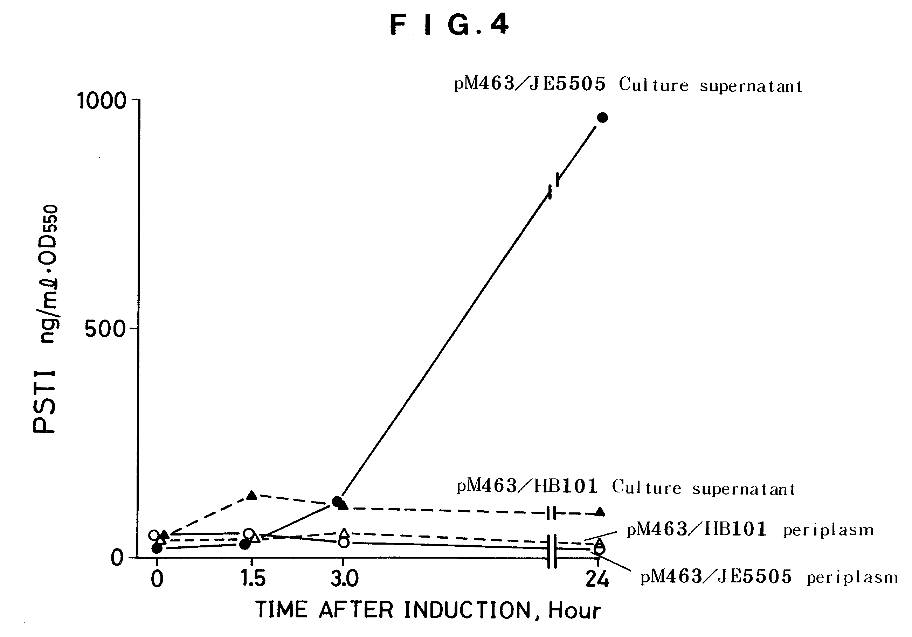

Further, pM463/HB101 and pM463/JE5505 strains were cultivated as described above, and the time-course of the amount of the human PSTI secreted to the culture supernatant and the periplasm were measured after the addition of 3-indoleacrylic acid. The results are shown in FIG. 4, wherein the amount of the human PSTI is expressed as a value obtained by dividing the measurement by turbidity in terms of absorbance ($OD_{550}$) representing the degree of propagation of the E. coli cell.

## Table 1   Expression of PSTI to Various Site

| Experiment | | PSTI (μg/ml medium) | | | β-galactosidase (mili unit/ml medium) | | |
|---|---|---|---|---|---|---|---|
| | | Supernatant | Periplasm | Cytoplasm | Supernatant | Periplasm | Cytoplasm |
| 1 | pM450/HB101 | <0.002 | <0.002 | 0.003 | −* | − | − |
| | pM450/JE5505 | <0.002 | <0.002 | 0.002 | − | − | − |
| | pM463/HB101 | 1.4 | 0.37 | <0.002 | − | − | − |
| | pM463/JE5505 | 5.9 | 0.031 | 0.55 | − | − | − |
| 2 | pM463/HB101 | 1.5 | 0.25 | 0.04 | 120 | 170 | 2700 |
| | pM463/JE5505 | 31 | 0.80 | 0.80 | 18 | 250 | 1300 |

* Not measured.

EP 0 300 459 A2

(2) Expression of human PSTI

pM463/JE5505 strain which has been cultivated overnight in L broth under shaking was innoculated into 50-fold volume of 100 µg/ml ampicillin-containing L broth, and cultivated overnight at 37° C under shaking. A 300 ml portion of the culture medium was centrifuged at 6,000 rpm for 5 minutes by a centrifuge 20PR-52 and a rotor RPR-20-2 both manufactured by Hitachi Koki K.K., and the resulting pellet was resuspended in 2 liters of 100 µg/ml ampicillin-containing L broth and cultivated at 37° C in a jar fermenter (Model MB-C manufactured by Iwashiya Bio-Science Co., Ltd.) until it reached its late-logarithmic growth phase. The culture medium was again centrifuged at 6,000 rpm for 10 minutes and the pellet was resuspended in 2 liters of 100 µg/ml ampicillin-containing M9CA medium. After cultivating at 37° C and adding 3-indoleacrylic acid to a final concentration of 40 µg/ml, it was further cultivated overnight. During the cultivation, the aeration rate, the agitation speed, and the pH were controlled to 1.5 to 3 liters/min., about 800 rpm, and about 7, respectively.

The thus obtained culture medium was centrifuged at 4° C and at 6,000 rpm for 5 minutes by using the rotor RPR-20-2 to remove the cells. An examination of the resulting culture supernatant by RIA revealed that 28 µg human PSTI was present per 1 ml supernatant.

Example 4 Preparation of anti-human PSTI monoclonal antibody

The human PSTI was partially purified as set forth below from the culture supernatant of the pM463/JE5505 strain obtained in Example 3(2).

A 300 ml aliquot of the culture supernatant prepared in Example 3(2) was dialyzed against 0.1N ammonium acetate buffer solution, pH 4.5 by using a dialysis membrane designed to fractionate molecules at a molecular weight of 3,500 manufactured by Spectrum Medical Industries Inc. and then concentrated by a concentrator (Diaflo YM2). The resulting concentrate was applied to a Sephadex G-50 column (diam. 2.5 cm x 100 cm) at a flow rate of 30 ml/hour. Human PSTI-positive fractions were collected by means of RIA detection, and used as an immunogen.

Next, a cell line producing the anti-human PSTI monoclonal antibody was isolated by a known method (T. Iwasaki et al, "Tan-Clone Sei Koutai to ELISA", Kodansha Scientific, 1983) as set forth below.

A 50 µl portion of the immunogen containing 120 µg human PSTI was mixed with 50 µl Freund's complete adjuvant (hereinafter abbreviated as FCA). The mixture was emulsified and administered subcutaneously, to a BALB/c mouse. An equal amount of the emulsion of the immunogen and the FCA was further administered intraperitonealy three times at two-week intervals. Spleen was extirpated four days after the final administration. Splenic cells isolated from the spleen were fused with mouse myeloma cell line P3U1 by using polyethylene glycol 1500. The fused cell population was cultivated in 100 µM hypoxanthine:0.4 µM aminopterin:16µM thymidine:10% fatal calf serum:RPMI1640 medium to select the fused cells from the splenic cells and the P3U1 cells.

The presence of the anti-human PSTI antibody in the culture supernatant of the selected cells were determined by enzyme linked immunosolbent assay (hereinafter abbreviated as ELISA) using a 96 well microplate in which the immunogen has been fixed to the bottom of each well. The cells which had been determined as positive in ELISA were mass-cultivated to prepare the anti-human PSTI monoclonal antibody as described below.

The cells which had been positive in ELISA was suspended in 2 liters of RPMI1640 medium supplmented with 5 µg/ml insulin, 5 µg/ml transferrin and, 0.1% bovine serum alubumin at a concentration of about $10^5$ cells/ml, and incubated at 37° C for 10 days. The culture medium was centrifuged at 8,000 rpm for 10 minutes. Ammonium sulfate was added to the resulting supernatant to a final concentration of 60%, and it was salted out at 4° C by agitating with a stirrer for 2 hours, and again centrifuged at 8,000 rpm at 4° C for 10 minutes. The resulting precipitate was dissolved in 0.1M phosphate buffer solution. The solution was applied to Protein A-Sepharose 4B column and eluted with 0.1M glycine-HCl buffer solution, pH 8.0 to obtain 47 mg monoclonal antibody.

The monoclonal antibody was immobilized on bromocyanactivated Sepharose 4B at about 1 mg/ml, and 3ml of the resin was poured into a column having a diameter of 11 mm. Into the thus prepared column, said

12

immunizing antigen, 0.1M sodium bicarbonate, pH 8.3-0.5N NaCl buffer solution, and 0.1M glycine-HCl buffer solution, pH 2.0 were sequentially passed. The eluate was monitored for the human PSTI by RIA, and it was recognized that the human PSTI was recovered in 0.1M glycine-HCl buffer solution, pH 2.0 to confirm a specific binding of the monoclonal antibody to the human PSTI.

Example 5 Purification of human PSTI

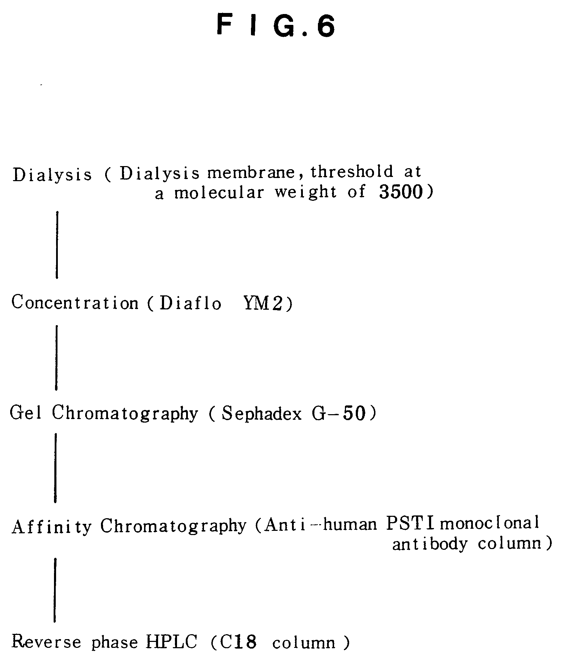

The human PSTI was purified from the culture supernatant of the pM463/JE5505 strain prepared in Example 3(2), as follows (FIG. 6).

A 850 ml portion of the culture supernatant prepared in Example 3(2) was dialyzed against 0.1N ammonium acetate buffer solution, pH 4.5 by using a dialysis membrane designed to fractionate molecules having a molecular weight of 3,500 manufactured by Spectrum Medical Industries Inc., and then concentrated by a concentrator (Diaflo YM2). The resulting concentrate was applied to a Sephadex G-50 column (diam. 2.5 cm x 100 cm), and eluted with 0.1N ammonium acetate buffer solution, pH 4.5 at a flow rate of 30 ml/hour. The eluate was monitored by RIA, and the fractions in which the human PSTI had been detected were aliquoted.

The aliquoted fractions were then concentrated in Diaflo YM2, dialyzed against physiological saline, and applied to a column filled with 25 ml of the Sepharose 4B having the anti-human PSTI monoclonal antibody immobilized thereto at 1 mg antibody/1 ml Sepharose 4B to effect affinity chromatography. The column was eluted with 0.1M glycine-HCl buffer solution, pH 2.0, and the fractions in which the human PSTI were detected by RIA were again concentrated by Diaflo YM2 and dialyzed against physiological saline. The dialysis concentrate was then applied to a reverse-phase HPLC using C18 columns. The column was developed with 0.01M ammonium acetate buffer solution, pH 4.5, and eluted with concentration gradient acetonitrile of 0 to 30%. Fractions corresponding to a peak detected at an absorbance of 280 nm eluting at approximately 28% acetonitrile were aliquoted.

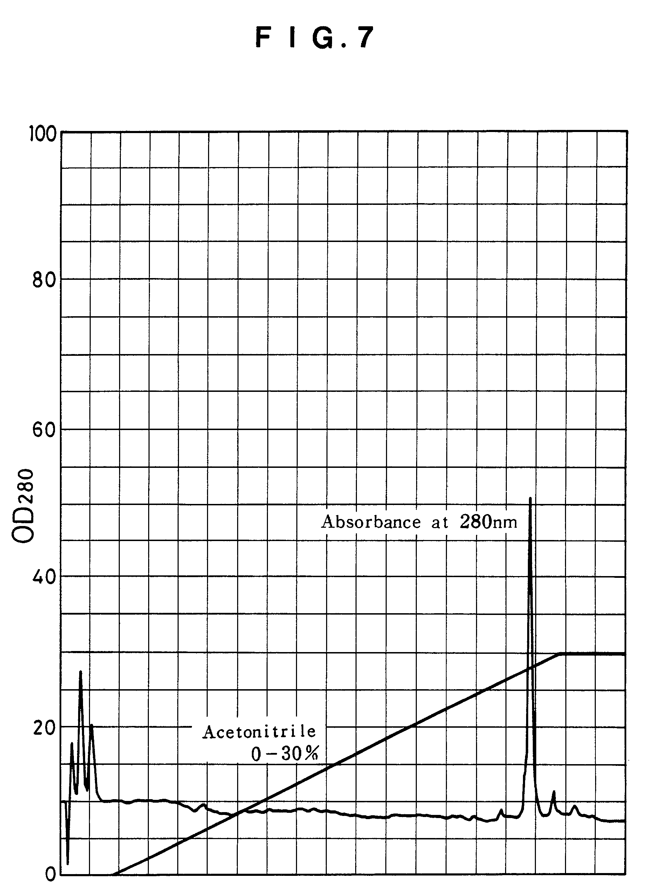

The elution profile from HPLC is shown in FIG. 7.

Example 6 Immunological identification of human PSTI

A 16 μl portion of the culture supernatant of the pM463/JE5505 strain obtained in Example 3(1) was subjected to an electrophoresis using 12.5% polyacrylamide gel modified with urea and SDS in accordance with a known method disclosed in Analytical Biochemistry, vol. 39, 462-477, 1971.

After completing the electrophoresis, proteins in the gel were immobilized onto a Nylon membrane Zeta-Probe manufactured by Bio-Rad Laboratories using a Semi Dry Electrobotter manufactured by Sartorius.

The Nylon membrane was incubated in 0.5M NaCl/20mM Tris-HCl pH7.5 buffer solution (hereinafter abbreviated as TS buffer solution) having 3% gelatin added thereto for 1 hour, and in a TS buffer solution containing 1% gelatin and 25 μg/ml mouse anti-human PSTI monoclonal antibody for 2 hours. The Nylon membrane was washed with distilled water and then washed twice with a TS buffer solution containing 0.05% Tween 20. The Nylon membrane was then immersed in a TS buffer solution containing 5 μg/ml horseradish peroxidase-conjugated goat antimouse immunoglobin antibody and 1% gelatin to allow for reaction for 1 hour. The Nylon membrane was again washed once with the distilled water, and twice with the TS buffer solution containing 0.05% Tween 20 for 10 minutes, and immersed in a TS buffer solution containing 5 mg/ml horseradish peroxidase color development manufactured by Bio-Rad Laboratories, 17% methanol and 0.015% hydrogen peroxide to allow for an enzyme reaction promoted by the horseradish peroxidase.

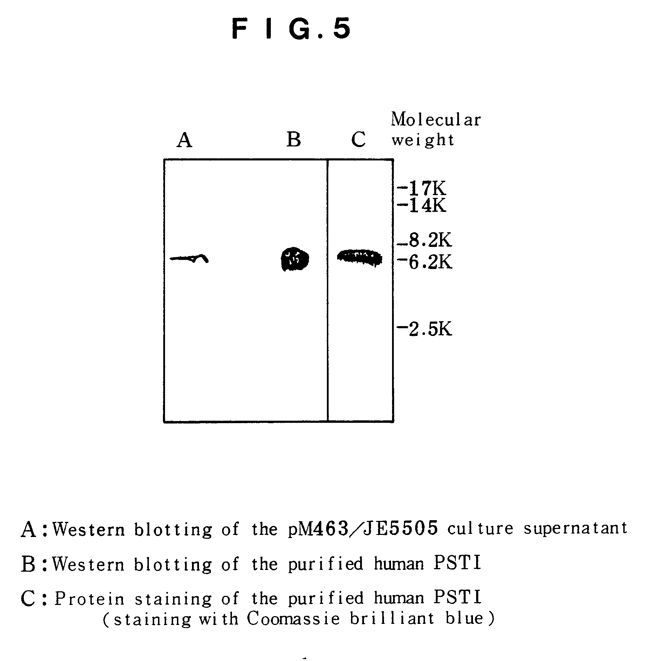

Band A which reacts with the anti-human PSTI monoclonal antibody was recognized at a position corresponding to a molecular weight of 6 kd by Western blotting method as shown in FIG. 5. This molecular weight conformed to the molecular weight calculated from the amino acid sequence of the human PSTI, 6.2 kd, but did not conform to the molecular weight calculated from the polypeptide including both the alkaline phosphatase signal peptide and the human PSTI, 8.5 kd.

Example 7 Trypsin inhibition activity of human PSTI

A 250 μl portion of the fraction aliquoted in Example 5 containing 9.65 μg/ml PSTI and its dilutions were respectively mixed with 500 μl 0.2M triethanolamine-HCl buffer solution and the mixtures were incubated at 25°C for 5 minutes. To this mixture, 50 μl 0.001N hydrochloric acid containing 90 μg/ml of trypsin (3120 NFU/mg, Novo Industrias) was added and incubated at 25°C for 5 minutes. 250 μl hydrochloric acid containing 1 mg/ml N-α-benzoyl-L-arginine-p-nitroanilide was added to the mixture and the mixture was incubated at 25°C for 10 minutes. When the reaction had been completed, 200 μl 50% acetic acid was added and absorbance at 405 nm was measured. Blank was prepared in a similar manner as above except for that 50 μl 0.001N hydrochloric acid was added instead of 50 μl trypsin.

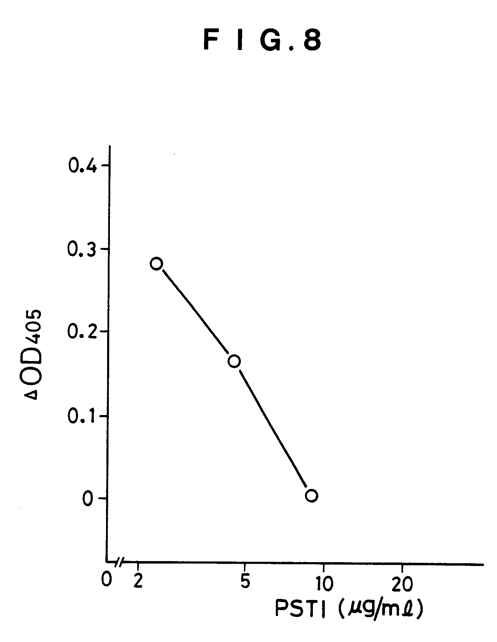

A trypsin inhibition curve of the culture supernatant of the pM463/JE5505 strain purified in Example 5 is plotted in FIG. 8 by measuring its absorbance.

The specific activity of the trypsin inhibition calculated from this result in accordance with a known method (M. Ogawa et al., Tan to Sui, vol.1, 1631-1638, 1980) was about 3,000 TIU/mg protein. This value was substantially consistent with the value of the natural human PSTI.

The amount of the proteins were measured by Lowry's method by using bovine serum albumin as a standatd.

Example 8 Protein-chemical properties of the purified human PSTI

The human PSTI purified in Example 5 was utilized in this example.

(A) Electrophoresis

A 0.2 μg and 2 μg portions of the human PSTI were respectively subjected to an electrophoresis using a 12.5% polyacrylamide gel modified with urea and SDS. The gel containing 0.2 μg human PSTI was subjected to the Western blotting in accordance with the method described in Example 6, and band B was obtained. The gel containing 2 μg human PSTI was stained with Coomassie Brilliant Blue, and the purified PSTI exhibited a single band C at a position corresponding to a molecular weight of about 6 kd. This position was consistent with the position of band A of Example 6 which resulted from the reaction with the anti-human PSTI monoclonal antibody and band B which resulted from the reaction between the 0.2 μg human PSTI and the anti-human PSTI monoclonal antibody (FIG. 5).

(B) Amino acid composition

A 160 μg portion of the human PSTI was reduced and carboxymethylated according to a known method (Method in Enzymology, vol. 11, E. H. W. Hirs ed., Academic Press, 1967), dialyzed against 0.01N hydrochloric acid, and dried under vacuum. It was then charged into PICO-TAG Work Station (Nihon Waters Ltd.) with 1% phenol/6N HCl. After sealing with nitrogen, the content was allowed to react at 110°C for 24, 48, or 72 hours to effect hydrolysis. The resulting hydrolysate was analyzed with a fully automated high-speed amino acid analyzer (Model A-5505 manufactured by Irika Kogyo K.K.) to obtain the analytical data shown in Table 2. The value for cystein is shown as a value for carboxymethylcysteine (CM-Cys) since the sample was subjected to the reduction and carboxymethylation. The results of the amino acid analysis shown in Table 2 were closely consistent with the theoretical value expected from the amino acid sequence of the human PSTI isoinhibitor B.

Table 2  Amino acid compositions of the purified human PSTI

| Amino acid | Mol % Measurement | Theoretical value | Amino acid | Mol % Measurement | Theoretical value |
|---|---|---|---|---|---|
| CM-Cys | 9.2 | 10.7 | Ile | 5.4 | 5.4 |
| Asx | 14.3 | 14.3 | Leu | 7.0 | 7.1 |
| Thr | 7.2 | 7.1 | Thy | 4.8 | 5.4 |
| Ser | 5.7 | 5.4 | Phe | 1.8 | 1.8 |
| Glx | 11.6 | 10 7 | Lys | 7.2 | 7.1 |
| Gly | 9.4 | 8.9 | His | 0 | 0 |
| Ala | 2.1 | 1.8 | Arg | 5.3 | 5.4 |
| Val | 3.1 | 3.6 | Pro | 5.8 | 5.4 |
| Met | 0 | 0 | Trp | * | 0 |

\* not measured

(C) Amino terminal amino acid sequence

A 13 μg portion of the human PSTI was subjected to Edman degradation by a known method (J. Biol. Chem. vol. 256, 7990-7997, 1981) using a gas-phase protein sequencer (470A, Applied Biosystems Inc.) to sequentially free the amino acid residues from the amino terminal as anilinothiazolinone derivatives and convert them to phenylthiohydantoin derivatives. The resulting phenylthiohydantoin derivatives were analyzed by PTH analyzer (120A, Applied Biosystems Inc.). The amino-terminal amino acid sequence of the human PSTI was identified from the amino terminal to the 22th amino acid except for 2 amino acids corresponding to half cystine. The result is shown as follows.

```
1                      5                          10
Asp-Ser-Leu-Gly-Arg-Glu-Ala-Lys-X-Tyr-

11                     15                         20
Asn-Glu-Leu-Asn-Gly-X-Thr-Lys-Ile-Tyr-

21
Asp-Pro-              X: unidentified
```

This sequence completely conformed to the amino acid sequence of the human PSTI which had been reported by N. Kikuchi et al. (J. Biochem. vol. 98, 687-694, 1985). Moreover, this implies that the alkaline phosphatase signal peptide connected to the human PSTI functioned to facilitate the permeation of the human PSTI through the cytoplasmic membrane in the cell of the pM463/JE5505, and that the connection between the signal peptide and the human PSTI was correctly cleaved.

(D) Carboxyl-terminal amino acid sequence

A 130 μg portion of the human PSTI was carboxymethylated as described in (C) of this Example, dialyzed against 50 mM ammonium bicarbonate, and lyophilized. It was then subjected to a hydrazinolysis by a known method ("Seikagaku Jikken Kouza 1 Tanpakushitsu no Kagaku", Japan Society of Biochemistry, Tokyo Kagaku Dojin. 1976), extracted with benzaldehyde, and the aqueous layer was vacuum dried.

An amino acid analysis was then carried out with a fully automated amino acid analyzer (Model A-5505 manufactured by Irika Kogyo K.K.) to detect the free amino acid. Carboxymethylcysteine was detected. This result indicates that the amino acid at the carboxy terminal of the human PSTI is cystein, which conformed to the report of N. Kikuchi et al. (J. Biochem. vol. 98, 687-694, 1985).

Example 9

Plasmids for expressing the human PSTI and transformants transformed with such plasmids were prepared in accordance with the process described in Examples 1, 2 or 3 except for that the expression promoter. SD sequence, signal peptide gene as well as some parts of the vectors and hosts used in these Examples were replaced with other types. The gene composition of the human PSTI-expression unit, the strain of E. coli used as host, the amount of the human PSTI expressed in culture supernatant are shown in Table 3. The transformants were cultivated as described below.

(a) Cultivation of the transformants including tryptophan operon promoter as a human PSTI-expression promoter

The process of Example 3(1) was repeated.

(b) Cultivation of the transformant including lactose operon promoter as a human PSTI-expression promoter

The process of Example 3(1) was repeated except for that isopropyl β-D-thiogalactopylanoside was added to a final concentration of 2 mM instead of 3-indoleacrylic acid.

(c) Cultivation of the transformant including lambda phage $P_L$ promoter as a human PSTI-expression promotor

An E. coli strain transformed with a vector having a temperature-sensitive mutant lambda phage $P_L$ promotor-represser gene (cl857) incorporated therein in addition to the human PSTI-expression unit was used. The process of Example 3(1) was repeated except for that the precultivation was effected at a temperature of about 30° C, and the temperature was raised to 42° C instead of adding 3-indoleacrylic acid and the cultivation was continued overnight at this temperature.

Abbreviations used in Table 3 are as follows.

amp: β-lactamase gene
trp: tryptophan gene
lac: lactose gene
lpp: lipoprotein gene
cll: lambda phage cll gene
$P_L$: lambda phage $P_L$ promoter
phoA: alkaline phosphatase gene

16

## Table 3

| Gene composition of the human PSTI-expression unit | | | | | Amount of human PSTI expressed in culture supernatant |
| Promoter | SD | Signal peptide | Vector | Host E. coli | (µg/ml) |
|---|---|---|---|---|---|
| trp | cII | phoA | pBR322 | JE5505 | 14 |
| trp | cII | phoA | pBR322 | HB101 | 1.5 |
| trp | cII | phoA | pBR322 | C600 | 0.07 |
| trp | cII | phoA | pBR322 | W3110 | 2.2 |
| trp | cII | phoA | pBR322 | SK107 | 0.06 |
| trp | cII | phoA | pBR322 | SG4044 | 2.9 |
| trp | cII | phoA | pBR322 | JE5513 | 11 |
| lac | cII | phoA | pBR322 | JE5505 | 4.4 |
| $P_L$ | cII | phoA | pBR322 | JE5505 | 3.4 |
| trp | trp | phoA | pBR322 | JE5505 | 10 |
| trp | lpp | lpp | pBR322 | JE5505 | 3.8 |
| trp | cII | amp | pBR322 | JE5505 | 4.1 |
| trp | cII | phoA | pACYC184 | JE5505 | 8.4 |

Example 10

Vectors including a DNA sequence encoding one of the human PSTI isoinhibitors A₁, A₂, and C, as well as the triptophan promoter, the lambda cII SD sequence, and the DNA sequence encoding the alkaline phosphatase signal peptide in the upstream of the human PSTI-encoding DNA sequence were prepared. The E. coli strain JE5505 was transformed by these vectors, and these transformants were then cultivated to produce the human PSTI isoinhibitors A₁, A₂ and C.

The vectors expressing the human PSTI isoinhibitors A₁, A₂ and C were prepared in accordance with the process of Example 1. More illustratively, a DNA including the DNA sequence encoding either of the human PSTI isoinhibitors A₁, A₂, and C, as well as the tryptophan operon promoter, the lambda cII SD sequence, and the DNA sequence encoding the alkaline phosphatase signal peptide in the upstream of the human PSTI-encoding DNA sequence were designed. These DNA sequences were respectively divided into 16 oligodeoxynucleotides, and these oligodeoxynucleotides were chemically synthesized in a fully automated DNA synthesizer, and purified by HPLC. The thus obtained oligodeoxynucleotides were phosphorylated with T4 polynucleotide kinase, respectively, and the 16 oligodeoxynucleotides were mixed and annealed to assemble the vector. A 264 bp double-stranded DNA fragment was constructed for the vector expressing the human PSTI isoinhibitor A₁, or A₂. A 249 bp double-stranded DNA fragment was constructed for the vector expressing the human PSTI isoinhibitor C.

An approximately 3.2 kbp DNA fragment obtained by digesting pM320 with HindIII and BamHI was isolated in accordance with the process of Example 1 by using the DEAE cellulose paper.

The above-described 264 bp or 249 bp double-stranded DNA fragment and the approximately 3.2 kbp DNA fragment derived from pM320 were assembled by T4 DNA ligase, and the resulting DNA was used to transform the JE5505 strain. Ampicillin resistant colonies were isolated to obtain the desired transformant, which were the transformant expressing the human PSTI isoinhibitor A₁, A₂ or C, respectively.

17

The thus prepared transformants expressing the human PSTI isoinhibitors A₁, A₂ and C, as well as the pM463/JE5505 strain were cultivated in accordance with the process of Example 3(1) to produce the human PSTI. The amount of the human PSTI in the culture supernatant was measured and the results are shown in Table 4.

## Table 4

| Isoinhibitor | Host | Human PSTI concentration in the culture supernatant (μg/ml) |
|---|---|---|
| A₁ | JE5505 | 6.3 |
| A₂ | JE5505 | 5.5 |
| B | JE5505 | 14 |
| C | JE5505 | 3.0 |

As apparent from the above-described results, production of the polypeptide having a set of properties conforming to that of the natural human PSTI was enabled by carrying out the present invention.

## EFFECT OF THE INVENTION

The human PSTI according to the present invention is essentially free of other proteins of human origin. Therefore, it can be processed into a variety of safe and uniform final products by selecting an appropriate isolation process depending on the intended purpose and use of the product.

The process for producing the human PSTI according to the present invention can advantageously produce a satisfactory amount of the human PSTI which is essentially free of other proteins of human origin.

The vectors and transformants according to the present invention may advantageously be used for producing the human PSTI which is essentially free of other proteins of human origin.

## Claims

1. A human pancreatic secretory trypsin inhibitor essentially free of other proteins of human origin.

2. The human pancreatic secretory trypsin inhibitor according to claim 1 prepared by transforming E. coli into a transformant capable of producing the human pancreatic secretory trypsin inhibitor (hereinafter abbreviated as PSTI), and cultivating the transformant to produce the human PSTI.

3. The human pancreatic secretory trypsin inhibitor according to claim 2 wherein said transformant is E. coli transformed with a vector replicable in E. coli and including a DNA sequence encoding human PSTI.

4. The human pancreatic secretory trypsin inhibitor according to claim 3 wherein said vector comprises both
   (A) a DNA sequence encoding the human PSTI, and
   (B) a promoter, an SD sequence, and a DNA sequence encoding a signal peptide, which function within E. coli.

5. The human pancreatic secretory trypsin inhibitor according to claim 3 or 4 wherein said vector is a plasmid designated pM463.

6. The human pancreatic secretory trypsin inhibitor according to any of the claims 2 to 5 wherein said E. coli has a mutation on its cell wall.

7. The human pancreatic secretory trypsin inhibitor according to any of the claims 2 to 6 wherein said E. coli is a strain designated JE5505 or JE5513.

8. The human pancreatic secretory trypsin inhibitor according to any of the claims 1 to 7 wherein said human PSTI is a protein comprising an amino acid sequence selected from

(a) Asp-Ser-Leu-Gly-Arg-Glu-Ala-Lys-Cys-Tyr-Asn-Glu-Leu-Asn-Gly-Cys-Thr-Lys-Ile-Tyr-Asp-Pro-Val-Cys-Gly-Thr-Asp-Gly-Asn-Thr-Tyr-Pro-Asn-Glu-Cys-Val-Leu-Cys-Phe-Glu-Asn-Arg-Lys-Arg-Gln-Thr-Ser-Ile-Leu-Ile-Gln-Lys-Ser-Gly-Pro-Cys,

(b) Asp-Ser-Leu-Gly-Arg-Glu-Ala-Lys-Cys-Tyr-Asn-Glu-Leu-Asn-Gly-Cys-Thr-Lys-Ile-Tyr-Asp-Pro-Val-Cys-Gly-Thr-Asp-Gly-Asn-Thr-Tyr-Pro-Asn-Glu-Cys-Val-Leu-Cys-Phe-Glu-Asn-Arg-Lys-Arg-Glu-Thr-Ser-Ile-Leu-Ile-Gln-Lys-Ser-Gly-Pro-Cys,

(c) Asp-Ser-Leu-Gly-Arg-Glu-Ala-Lys-Cys-Tyr-Asn-Glu-Leu-Asn-Gly-Cys-Thr-Lys-Ile-Tyr-Asp-Pro-Val-Cys-Gly-Thr-Asp-Gly-Asn-Thr-Tyr-Pro-Asn-Glu-Cys-Val-Leu-Cys-Phe-Glu-Asn-Arg-Lys-Arg-Gln-Thr-Ser-Ile-Leu-Ile-Glu-Lys-Ser-Gly-Pro-Cys, and

(d) Glu-Ala-Lys-Cys-Tyr-Asn-Glu-Leu-Asn-Gly-Cys-Thr-Lys-Ile-Tyr-Asp-Pro-Val-Cys-Gly-Thr-Asp-Gly-Asn-Thr-Tyr-Pro-Asn-Glu-Cys-Val-Leu-Cys-Phe-Glu-Asn-Arg-Lys-Arg-Gln-Thr-Ser-Ile-Leu-Ile-Gln-Lys-Ser-Gly-Pro-Cys.

9. A process for producing a human pancreatic secretory trypsin inhibitor comprising transforming E. coli into a transformant capable of producing the human PSTI, cultivating said transformant to produce the human PSTI, and recovering the human PSTI from the culture supernatant.

10. The process according to claim 9 wherein said transformant is E. coli transformed with a vector replicable in E. coli and including a DNA sequence encoding the human PSTI.

11. The process according to claim 10 wherein said vector comprises both

(A) a DNA sequence encoding the human PSTI, and

(B) a promoter, an SD sequence, and a DNA sequence encoding a signal peptide, which function within E. coli.

12. The process according to claim 10 or 11 wherein said vector is a plasmid designated pM463.

13. The process according to any of the claims 9 to 12 wherein said E. coli has a mutation on its cell wall.

14. The process according to any of the claims 9 to 13 wherein said E. coli is a strain designated JE5505 or JE5513.

15. The process according to any of the claims 9 to 14 wherein said human PSTI is a protein comprising an amino acid sequence selected from

(a) Asp-Ser-Leu-Gly-Arg-Glu-Ala-Lys-Cys-Tyr-Asn-Glu-Leu-Asn-Gly-Cys-Thr-Lys-Ile-Tyr-Asp-Pro-Val-Cys-Gly-Thr-Asp-Gly-Asn-Thr-Tyr-Pro-Asn-Glu-Cys-Val-Leu-Cys-Phe-Glu-Asn-Arg-Lys-Arg-Gln-Thr-Ser-Ile-Leu-Ile-Gln-Lys-Ser-Gly-Pro-Cys,

(b) Asp-Ser-Leu-Gly-Arg-Glu-Ala-Lys-Cys-Tyr-Asn-Glu-Leu-Asn-Gly-Cys-Thr-Lys-Ile-Tyr-Asp-Pro-Val-Cys-Gly-Thr-Asp-Gly-Asn-Thr-Tyr-Pro-Asn-Glu-Cys-Val-Leu-Cys-Phe-Glu- Asn-Arg-Lys-Arg-Glu-Thr-Ser-Ile-Leu-Ile-Gln-Lys-Ser-Gly-Pro-Cys,

(c) Asp-Ser-Leu-Gly-Arg-Glu-Ala-Lys-Cys-Tyr-Asn-Glu-Leu-Asn-Gly-Cys-Thr-Lys-Ile-Tyr-Asp-Pro-Val-Cys-Gly-Thr-Asp-Gly-Asn-Thr-Tyr-Pro-Asn-Glu-Cys-Val-Leu-Cys-Phe-Glu-Asn-Arg-Lys-Arg-Gln-Thr-Ser-Ile-Leu-Ile-Glu-Lys-Ser-Gly-Pro-Cys, and

(d) Glu-Ala-Lys-Cys-Tyr-Asn-Glu-Leu-Asn-Gly-Cys-Thr-Lys-Ile-Tyr-Asp-Pro-Val-Cys-Gly-Thr-Asp-Gly-Asn-Thr-Tyr-Pro-Asn-Glu-Cys-Val-Leu-Cys-Phe-Glu-Asn-Arg-Lys-Arg-Gln-Thr-Ser-Ile-Leu-Ile-Gln-Lys-Ser-Gly-Pro-Cys.

16. A vector replicable in E. coli comprising

(A) a DNA sequence encoding the human PSTI, and

19

(B) a promoter, an SD sequence, and a DNA sequence encoding a signal peptide, which function within E. coli.

17. The vector according to claim 16 wherein said E. coli has a mutation on its cell wall.

18. The vector according to claim 16 or 17 wherein said E. coli is a strain designated JE5505 or JE5513

19. The vector according to any of the claims 16 to 18 wherein said vector is a plasmid designated pM463.

20. The vector according to any of the claims 16 to 19 wherein said human PSTI is a protein comprising an amino acid sequence selected from

(a) Asp-Ser-Leu-Gly-Arg-Glu-Ala-Lys-Cys-Tyr-Asn-Glu-Leu-Asn-Gly-Cys-Thr-Lys-Ile-Tyr-Asp-Pro-Val-Cys-Gly-Thr-Asp-Gly-Asn-Thr- Tyr-Pro-Asn-Glu-Cys-Val-Leu-Cys-Phe-Glu-Asn-Arg-Lys-Arg-Gln-Thr-Ser-Ile-Leu-Ile-Gln-Lys-Ser-Gly-Pro-Cys,

(b) Asp-Ser-Leu-Gly-Arg-Glu-Ala-Lys-Cys-Tyr-Asn-Glu-Leu-Asn-Gly-Cys-Thr-Lys-Ile-Tyr-Asp-Pro-Val-Cys-Gly-Thr-Asp-Gly-Asn-Thr-Tyr-Pro-Asn-Glu-Cys-Val-Leu-Cys-Phe-Glu-Asn-Arg-Lys-Arg-Glu-Thr-Ser-Ile-Leu-Ile-Gln-Lys-Ser-Gly-Pro-Cys,

(c) Asp-Ser-Leu-Gly-Arg-Glu-Ala-Lys-Cys-Tyr-Asn-Glu-Leu-Asn-Gly-Cys-Thr-Lys-Ile-Tyr-Asp-Pro-Val-Cys-Gly-Thr-Asp-Gly-Asn-Thr-Tyr-Pro-Asn-Glu-Cys-Val-Leu-Cys-Phe-Glu-Asn-Arg-Lys-Arg-Gln-Thr-Ser-Ile-Leu-Ile-Glu-Lys-Ser-Gly-Pro-Cys, and

(d) Glu-Ala-Lys-Cys-Tyr-Asn-Glu-Leu-Asn-Gly-Cys-Thr-Lys-Ile-Tyr-Asp-Pro-Val-Cys-Gly-Thr-Asp-Gly-Asn-Thr-Tyr-Pro-Asn-Glu-Cys-Val-Leu-Cys-Phe-Glu-Asn-Arg-Lys-Arg-Gln-Thr-Ser-Ile-Leu-Ile-Gln-Lys-Ser-Gly-Pro-Cys.

21. A transformant of E. coli transformed by a vector replicable in E. coli comprising

(A) a DNA sequence encoding the human PSTI, and

(B) a promoter, an SD sequence, and a DNA sequence encoding a signal peptide, which function within E. coli.

22. The transformant of E. coli according to claim 21 wherein said E. coli has a mutation on its cell wall.

23. The transformant of E. coli according to claim 21 or 22 wherein said E. coli is a strain designated JE5505 or JE5513.

24. The transformant of E. coli according to any of the claims 21 to 23 wherein said vector is a plasmid designated pM463.

25. The transformant of E. coli according to any of the claims 21 to 24 wherein said human PSTI is a protein comprising an amino acid sequence selected from

(a) Asp-Ser-Leu-Gly-Arg-Glu-Ala-Lys-Cys-Tyr-Asn-Glu-Leu-Asn-Gly-Cys-Thr-Lys-Ile-Tyr-Asp-Pro-Val-Cys-Gly-Thr-Asp-Gly-Asn-Thr-Tyr-Pro-Asn-Glu-Cys-Val-Leu-Cys-Phe-Glu-Asn-Arg-Lys-Arg-Gln-Thr-Ser-Ile-Leu-Ile-Gln-Lys-Ser-Gly-Pro-Cys,

(b) Asp-Ser-Leu-Gly-Arg-Glu-Ala-Lys-Cys-Tyr-Asn-Glu-Leu-Asn-Gly-Cys-Thr-Lys-Ile-Tyr-Asp-Pro-Val-Cys-Gly-Thr-Asp-Gly-Asn-Thr-Tyr-Pro-Asn-Glu-Cys-Val-Leu-Cys-Phe-Glu-Asn-Arg-Lys-Arg-Glu-Thr-Ser-Ile-Leu-Ile-Gln-Lys-Ser-Gly-Pro-Cys,

(c) Asp-Ser-Leu-Gly-Arg-Glu-Ala-Lys-Cys-Tyr-Asn-Glu-Leu-Asn-Gly-Cys-Thr-Lys-Ile-Tyr-Asp-Pro-Val-Cys-Gly-Thr-Asp-Gly-Asn-Thr-Tyr-Pro-Asn-Glu-Cys-Val-Leu-Cys-Phe-Glu-Asn-Arg-Lys-Arg-Gln-Thr-Ser-Ile-Leu-Ile-Glu-Lys-Ser-Gly-Pro-Cys, and

(d) Glu-Ala-Lys-Cys-Tyr-Asn-Glu-Leu-Asn-Gly-Cys-Thr-Lys-Ile-Tyr-Asp-Pro-Val-Cys-Gly-Thr-Asp-Gly-Asn-Thr-Tyr-Pro-Asn-Glu-Cys-Val-Leu-Cys-Phe-Glu-Asn-Arg-Lys-Arg-Gln-Thr-Ser-Ile-Leu-Ile-Gln-Lys-Ser-Gly-Pro-Cys.

S01                                                S03                                          60
AGCTTATCTAAGGAAATACTTACATATGG|ACTCCCTAGGTCGCGAGGCCAAATGTTACAA
       ATAGATTCCTTTATGAATGTATACCTGAGGGA|TCCAGCGCTCCGGTTTACAATGTT
       λcⅡSD                                          S02

                    f−met AspSerLeuGlyArgGluAlaLysCysTyrAs


       S05                                                S07                                      120
TG|AACTTAATGGATGCACCAAGATATATGACCCTGT|CTGTGGGACTGATGGAAATACTTA
ACTTGAATTA|CCTACGTGGTTCTATATACTGGGACAGACACCCT|GACTACCTTTATGAAT
       S04                                                S06

nGluLeuAsnGlyCysThrLysIleTyrAspProValCysGlyThrAspGlyAsnThrTy


             S09                                           S11              180
TCCCAATGAAT|GCGTGTTATGTTTTGAAAATCGGAAACGCCAGAC|ATCGATCCTCATTCA
AGGGTTACTTACGCACAA|TACAAAACTTTTAGCCTTTGCGGTCTGTAGCTAG|GAGTAAGT
             S08                                           S10


rProAsnGluCysValLeuCysPheGluAsnArgLysArgGlnThrSerIleLeuIleGl



AAAATCTGGGCCTTGCTGAG
TTTTAGACCCGGAACGACTCCTAG
                    S12

nLysSerGlyProCys          **F I G. 1**

EP 0 300 459 A2

# F I G . 2

# F I G. 3a

S15
AGCTTATCTAAGGAAATACTTAAAAATGAAAC
ATAGATTCCTTTATGAATTTTTACTTTGTTTCGTGA
S16

S17
AAAGCACTATTGCACTGGCACTCTTACCGTTACTGTTT
TAACGTGACCGTGAGAATGGCAATGACAAATGGGGA
S18

S19
ACCCCTGTGACAAAAGCCGACTCCCTAGGTCG
CACTGTTTTCGGCTGAGGGATCCAGC
S20

EcoRI

trp
Hind III
Nde I
Nru I

Ap^r
pM450
3.4Kb

BamHI

Kination
Annealing
Ligation

Alkaline phosphatase signal peptide

AGCTTATCTAAGGAAATACTTAAAAATGAAACAAAGCACTATTGCACTGGCACTCTTACC
ATAGATTCCTTTATGAATTTTTACTTTGTTTCGTGATAACGTGACCGTGAGAATGG

Hind III

PSTI

GTTACTGTTTACCCCTGTGACAAAAGCCGACTCCCTAGGTCG
CAATGACAAATGGGGACACTGTTTTCGGCTGAGGGATCCAGC

Nru I

Double digestion with
Hind III, Nru I

EcoRI

Hind III
Nru I
Ap^r
Ban III
BamHI

Ligation

EcoRI

trp
Hind III
phoA SP
Nru I
Ap^r
pM463
3.5Kb
PSTI
Ban III
BamHI

EP 0 300 459 A2

# F I G. 3b

EP 0 300 459 A2

```
        10          20          30          40          50          60
ATGAAACAAAGCACTATTGCACTGGCACTCTTACCGTTACTGTTTACCCCTGTGACAAAA
MetLysGlnSerThrIleAlaLeuAlaLeuLeuProLeuLeuPheThrProValThrLys
←───── alkaline phosphatase signal peptide        ────
        70          80          90         100         110         120
GCCGACTCCCTAGGTCGCGAGGCCAAATGTTACAATGAACTTAATGGATGCACCAAGATA
AlaAspSerLeuGlyArgGluAlaLysCysTyrAsnGluLeuAsnGlyCysThrLysIle
→→←────── PSTI
       130         140         150         160         170         180
TATGACCCTGTCTGTGGGACTGATGGAAATACTTATCCCAATGAATGCGTGTTATGTTTT
TyrAspProValCysGlyThrAspGlyAsnThrTyrProAsnGluCysValLeuCysPhe

       190         200         210         220         230
GAAAATCGGAAACGCCAGACATCGATCCTCATTCAAAAATCTGGGCCTTGCTGA
GluAsnArgLysArgGlnThrSerIleLeuIleGlnLysSerGlyProCys***
                                              →────────→
```

# F I G . 4

pM463/JE5505 Culture supernatant

pM463/HB101 Culture supernatant

pM463/HB101 periplasm

pM463/JE5505 periplasm

PSTI ng/ml·OD550

1000

500

0

0    1.5    3.0    24

TIME AFTER INDUCTION, Hour

# F I G. 5

A:Western blotting of the pM463/JE5505 culture supernatant

B:Western blotting of the purified human PSTI

C:Protein staining of the purified human PSTI
(staining with Coomassie brilliant blue)

# F I G. 6

Dialysis ( Dialysis membrane, threshold at
                a molecular weight of **3500** )

Concentration ( Diaflo  YM2 )

Gel Chromatography ( Sephadex G−50 )

Affinity Chromatography (Anti−human PSTI monoclonal
                                antibody column )

Reverse phase HPLC (C18  column )

# F I G . 7

# F I G . 8